# FASCICULE DE BREVET EUROPEEN

(11) **EP 3 325 606 B1**
(45) Date de publication et mention de la délivrance du brevet: **05.10.2022**
(21) Numéro de dépôt: 16754404.8
(22) Date de dépôt: 13.07.2016
(51) Int. Cl.: C12N 1/12, C12P 21/00

(54) **BIOMASSE DE THRAUSTOCHYTRIDES ENRICHIE EN HUILES ET EN PROTEINES, PROCEDE DE CULTURE ET UTILISATIONS**
MIT ÖLEN UND PROTEINEN ANGEREICHERTE THRAUSTOCHYTRIUM-BIOMASSE, KULTURVERFAHREN UND VERWENDUNGEN
THRAUSTOCHYTRID BIOMASS ENRICHED WITH OILS AND PROTEINS, CULTURING METHOD, AND USES

(30) Priorité: 17.07.2015 FR 1556792
(43) Date de publication de la demande: 30.05.2018
(73) Titulaire: Fermentalg, 33500 Libourne (FR)
(72) Inventeur: CAGNAC, Olivier, 33500 Libourne (FR); PAGLIARDINI, Julien, 33000 Bordeaux (FR); CALLEJA, Pierre, 33450 Saint Sulpice et Cameyrac (FR); LEPENDRIE, Adeline, 33500 Libourne (FR)
(74) Mandataire: Be IP
(86) Numéro de dépôt international: PCT/EP2016/066594
(87) Numéro de publication internationale: WO 2017/012933

(56) Documents cités:
- WO-A1-2012/175027
- WO-A1-2015/079182
- WO-A1-2016/120558
- WO-A2-2015/004402
- WO-A2-2015/004403
- US-A1- 2009 209 014
- US-A1- 2010 239 533
- SUN LINA ET AL: "Differential effects of nutrient limitations on biochemical constituents and docosahexaenoic acid production ofSchizochytriumsp", BIORESOURCE TECHNOLOGY, vol. 159, 4 mars 2014 (2014-03-04), pages 199-206, XP028646306, ISSN: 0960-8524, DOI: 10.1016/J.BIORTECH.2014.02.106
- YAMASAKI T ET AL: "Utilization of Shochu distillery wastewater for production of polyunsaturated fatty acids and xanthophylls using thraustochytrid", JOURNAL OF BIOSCIENCE AND BIOENGINEERING, ELSEVIER, AMSTERDAM, NL, vol. 102, no. 4, 1 octobre 2006 (2006-10-01), pages 323-327, XP028042220, ISSN: 1389-1723, DOI: 10.1263/JBB.102.323 [extrait le 2006-10-01]
- LIU YING ET AL: "Culturable diversity and biochemical features of thraustochytrids from coastal waters of Southern China", APPLIED MICROBIOLOGY AND BIOTECHNOLOGY, SPRINGER, DE, vol. 98, no. 7, 24 novembre 2013 (2013-11-24), pages 3241-3255, XP035329079, ISSN: 0175-7598, DOI: 10.1007/S00253-013-5391-Y [extrait le 2013-11-24]
- Kousoulaki K.: "Heterotrophic Microalgae for Omega-3 Rich Fish: Atlantic Salmon Performance, Health and Product Quality", International Symposium on Fish Nutrition and Feeding , 30 mai 2014 (2014-05-30), page 18, XP055150488, Extrait de l'Internet: URL:http://www.protix.eu/wp-content/upload s/2014/07/ISFNF14-Proceedings.pdf [extrait le 2014-11-03]

## Description

### DOMAINE DE L'INVENTION

L'invention se place dans le domaine des cultures de microalgues particulièrement des Thraustochytrides. Elle a pour objet une biomasse de Thraustochytrides riche en protéines et en lipides, et éventuellement comprenant des caroténoïdes, son procédé d'obtention et ses utilisations dans l'alimentation.

### ETAT DE LA TECHNIQUE

On connaît plusieurs sources de protéines et des sources de lipides d'origine végétale pour leur utilisation dans l'alimentation humaine ou animale, directement ou comme compléments alimentaires, pour apporter aux animaux et humains des acides aminés nécessaires à leur métabolisme. Par "sources de protéines", on entend des sources d'acides aminés disponibles pour les animaux ou humains une fois les aliments ingérés. Les lipides, en particulier les acides gras polyinsaturés ("polyunsaturated fatty acids en anglais" ou PUFAs), ont un intérêt en tant que source d'énergie, ainsi que pour leurs propriétés thérapeutiques. Parmi les acides gras polyinsaturés, certains hautement insaturés (AGHI) de la série des oméga-3 (PUFA-ω3), en particulier, l'acide éicosapentaénoïque (EPA ou C20 :5 ω3) et l'acide docosahexaénoïque (DHA ou C22 :6 ω3), et de la série des oméga-6 (PUFA-ω6), en particulier, l'acide arachidonique (ARA ou AA ou encore acide eicosatétraénoïque C20 :4 ω6) ont une importance nutritionnelle reconnue et présentent de fortes potentialités en terme d'applications thérapeutiques. Il serait donc souhaitable de pouvoir fournir des nouvelles sources de protéines et de PUFAs pour leur utilisation dans l'alimentation animale et humaine. Des sources qui contiennent des protéines ainsi que des lipides, en particulier, des PUFAs, présenteraient des avantages considérables.

Comme sources de protéines, on connaît la farine de poissons, sous-produit de la pêche, relativement riche en protéines mais pauvre en lipides (on peut citer des teneurs d'environ 8% en matière sèche)

La plus connue des sources de protéines végétales employée dans l'alimentation animale est le soja, généralement employé sous forme de tourteaux, résidu solide restant après extraction de l'huile. Toutefois, l'emploi de tourteaux de soja présente plusieurs inconvénients associés à leur origine, notamment de variétés de soja génétiquement modifiées (OGM),. En outre, le soja, comme de nombreuses sources de protéines végétales, n'est pas une source de lipides tels que des PUFAs.

On connaît d'autres sources de protéines végétales, notamment la spiruline ou la chlorelle, employées comme compléments alimentaires chez l'homme.

La spiruline, comme la chlorelle, présentent toutefois l'inconvénient d'une faible productivité en protéine qui ne permet pas le passage à une culture en fermenteur à rendement élevé. Leur culture ne permet pas de répondre à un objectif de production industrielle plus large, économiquement viable, d'une source de protéines alimentaires dont les qualités lui permettront de remplacer les sources usuelles comme le soja dans l'alimentation des animaux et de l'homme. De plus, la chlorelle et la spiruline ont un contenu très faible en PUFAs.

Comme les protéines et les lipides, les caroténoïdes présentent également un intérêt dans les nutritions animale et humaine.

Souvent, la production de ces pigments à grande échelle demande une main d'oeuvre intensive et de grandes surfaces de production..

Par ailleurs aujourd'hui, aucune des sources de protéines, ni d'acides gras citées, ne contient des quantités importantes de caroténoïdes.

De nouvelles sources de caroténoïdes, notamment de lutéine, de fucoxanthine, d'astaxanthine, de zéaxanthine, de cantaxanthine, d'échinénone, de bêta-carotènes et de phoenicoxanthine doivent donc être recherchées afin de répondre à la demande croissante du marché pour ces molécules.

Les protistes connus pour répondre à une capacité de production industrielle en fermenteurs, sont employés depuis longtemps pour la production de matières grasses riches en acides gras polyinsaturés comme le DHA ou l'EPA, par exemple WO 97/37032, WO 2015/004402 ou WO 2012/17027. Certains protistes sont capables, sous certaines conditions, de produire des caroténoïdes. Cependant, les biomasses obtenues jusqu'à maintenant, y compris après extraction des matières grasses, ne contenaient pas de teneurs suffisantes en protéines pour permettre leur usage comme source de protéines dans l'alimentation, pour le moins sans étapes additionnelles d'enrichissement en protéines économiquement coûteuses.

L'un des buts que vise l'invention est de fournir une nouvelle source de protéines et de lipides, en particulier des PUFAs pour l'alimentation animale ou humaine répondant à un objectif de production industrielle large, économiquement viable. Cette source de protéines et de PUFAs peut également comprendre des caroténoïdes, en particulier, des carotènes (α, β, ε, γ, δ ou ζ-carotène, lycopène et phytoène) et les xanthophylles (astaxanthine, anthéraxanthine, citranaxanthine, cryptoxanthine, canthaxanthine, diadinoxanthine, diatoxanthine, flavoxanthine, fucoxanthine, lutéine, néoxanthine, phoenicoxanthine, rhodoxanthine, rubixanthine, siphonaxanthine, violaxanthine, zéaxanthine).

L'invention montre que dans certaines conditions de culture, les Thraustochytrides, connus pour leur utilisation dans la production d'huiles à fortes teneurs en acides gras polyinsaturés (DHA et EPA, notamment) sont des microorganismes aptes à produire une grande quantité de protéines, pour l'alimentation animale et une source protéique de haute valeur pour la nutrition humaine. Les inventeurs, ont pu déterminer des conditions de culture permettant l'obtention d'une forte teneur en protéines tout en gardant une teneur en lipides d'au moins 20% (par rapport à la matière sèche), dont 25-60%, de PUFAs. Ils ont aussi pu déterminer des conditions de culture permettant d'orienter des cellules vers la production des caroténoïdes en plus des protéines et des PUFAs.

### EXPOSE DE L'INVENTION

L'invention concerne un procédé de préparation d'une biomasse de Thraustochytrides dont une quantité substantiellement majoritaire n'est pas dégradée, comprenant des protéines et des acides gras, caractérisée en ce qu'elle comprend en poids par rapport au poids de la matière sèche, au moins 40% de protéines et au moins 20%, de matière grasse, la matière grasse ayant une teneur de 25 à 60% en acides gras polyinsaturés choisi parmi l'acide arachidonique (AA), l'acide docosahexaénoïque (DHA) et l'acide éicosapentaénoïque (EPA), sans protéines ni acides gras ajoutés, caractérisé en ce qu'il comprend :
a. une première étape de culture des Thraustochytrides réalisée en mode dit "batch", en mode dit "fed batch" ou en mode continu dans des conditions de hétérotrophie ou de mixotrophie dans un milieu de culture défini comprenant une source de carbone, une source d'azote, une source de phosphore et des sels, à une température comprise entre environ 24°C et 35°C et dans des conditions pour favoriser la production de protéines à une teneur d'au moins 40% de protéines en poids par rapport au poids de la matière sèche,
   l'étape a) étant divisée en deux sous-étapes, une première sous-étape a1) de croissance dans le milieu de culture approprié jusqu'à obtenir des teneurs en source de carbone dans le milieu inférieure à 20 g/L, suivie d'une deuxième sous-étape a2) de production dans laquelle on ajoute au milieu de culture, simultanément ou successivement, une ou plusieurs solution(s) d'enrichissement en source de carbone, en source d'azote et en source de phosphore , la teneur en source de carbone est maintenue entre 0 et 50 g/L, la teneur en source d'azote est maintenue entre 0,5 et 5 g/L, et la teneur en source de phosphore est maintenue entre 0,5 et 5 g/L ;
b. une deuxième étape de culture dans des conditions de hétérotrophie ou de mixotrophie à une température entre environ 18°C et 25°C et inférieure à celle de l'étape a) dans des conditions pour favoriser l'accumulation de matière grasse jusqu'à au moins 20% en poids par rapport au poids de la matière sèche, et jusqu'à l'obtention d'une densité de culture d'au moins 40 g/L en matière sèche ;
c. une troisième étape de récupération de la biomasse obtenue à la deuxième étape par séparation de ladite biomasse du milieu de culture (la récolte), ; et, le cas échéant
d. une quatrième étape de séchage de la biomasse récupérée à la troisième étape.

L'invention concerne également une biomasse de Thraustochytrides pouvant comprendre, en poids par rapport au poids de la matière sèche, au moins 40% de protéines, voire plus de 60% de protéines en poids par rapport au poids de la matière sèche et au moins 20% de matière grasse, préférentiellement au moins 30% de matière grasse, la matière grasse avant une teneur de 25 à 60%, de préférence, entre 40% et 60% en acides gras polvinsaturés (PUFAs) choisi parmi l'acide arachidoniaue (AA), l'acide docosahexaénoïque (DHA) et l'acide éicosapentaénoïque (EPA), sans protéines ni acides gras ajoutés, susceptible d'être obtenue par le procédé selon l'invention. La biomasse peut contenir, de manière générale, entre 40% et 65% de protéines, en particulier, de 45% à 55% de protéines par rapport au poids de la matière sèche.

Les pourcentages en poids en protéines peuvent être exprimés tant par rapport aux protéines elles-mêmes que par rapport aux acides aminés contenus dans lesdites protéines. Les pourcentages indiqués ici sont calculés par la somme des acides aminés totaux contenus dans la biomasse (norme ISO 13903 :2005) et non pas à partir de l'azote total. Par exemple, un analyseur d'acides aminés peut être utilisé pour mesurer les acides aminés totaux. Le tryptophane est dosé à part, sa valeur est ensuite additionnée à la somme des autres acides aminés pour obtenir la somme des acides aminés totaux.

Selon un mode de réalisation de l'invention, le DHA peut être le PUFA préféré.

Selon certains modes de réalisation de l'invention, ladite biomasse peut comprendre également entre 5 et 250 ppm (ng/mg de matière sèche), de préférence entre 150 et 200 ppm de caroténoïdes.

Le temps de culture peut-être, de manière générale, compris entre 40 et 100 heures, de préférence entre 65 et 96 heures, par exemple, 70, 84 ou 96 heures. La méthode de culture objet de l'invention peut permettre de manière générale d'atteindre une concentration en biomasse supérieure à 100 g/L, voire 120 g/L.

Dans la première étape a), les cellules peuvent être cultivées pendant environ 18-30 heures, de préférence pendant 24h. Après cette première étape de culture, par exemple, à partir d'environ 24h de culture, la température peut être abaissée à environ 18-24°C (pour démarrer l'étape b)). Cette baisse en température peut favoriser la production des acides gras et éventuellement des caroténoïdes. La culture à basse température peut être en général poursuivie jusqu'à entre environ 50 et 70 heures de culture, et en tout cas de préférence, jusqu'à la fin de la culture.

On peut éventuellement induire la production des caroténoïdes pendant la culture, de préférence, pendant l'étape b). Pour cela, des conditions mixotrophiques avec une lumière, de préférence, bleue à environ 435-475nm, de préférence 455 nm, peuvent être appliquées. Des caroténoïdes tels que l'astaxanthine, la canthaxanthine, le bêta-carotène et la phoenicoxanthine, peuvent être produits avec ces longueurs d'onde. La lumière peut être, de manière générale, appliquée pour au moins 18 heures, de préférence pour au moins 24 heures.

Selon un mode de réalisation préféré de l'invention, la lumière peut être appliquée à la culture à partir de 24 à 50 heures, plus préférentiellement, à partir de 45 heures de culture et jusqu'à la fin de la culture.

L'invention concerne aussi l'utilisation d'une biomasse telle que ci-dessus ou ci-après, dans les domaines de l'alimentation humaine ou animale, notamment en aquaculture. L'invention concerne aussi l'utilisation d'une biomasse telle que ci-dessus ou ci-après, pour l'amélioration de la performance des animaux. Ladite performance peut être évaluée par la mesure de l'indice de consommation, du gain de poids ou encore du "Feed Conversion Ratio", tous connus à l'homme de métier.

L'invention concerne aussi un aliment comprenant une telle biomasse qui permet à la fois d'augmenter sa valeur et sa densité nutritionnelle.

L'invention concerne également la biomasse selon l'invention pour son utilisation en thérapie, notamment dans le traitement et dans la prévention de la malnutrition.

### DESCRIPTION DETAILLEE DE L'INVENTION

Par "mutants", on entend un organisme dérivé de la souche originelle dont le patrimoine génétique a été modifié soit par un processus naturel, soit par des méthodes physico-chimiques connues par l'homme de l'art pouvant générer des mutations aléatoires (UV etc.), soit par des méthodes de génie génétique.

Par "conditions de mixotrophie" ou par "en mode mixotrophe", on entend une culture avec un apport de lumière et un apport en substrat carboné organique et dans l'obscurité.

Par "conditions de mixotrophie à dominante hétérotrophe", on entend des conditions d'illumination dans lequel la majeure partie de l'énergie pour la croissance des cellules est fourni par une source de carbone organique, mais dans lequel la lumière a un impact sur la croissance et / ou la composition des cellules. Cette lumière peut être fournie soit en continu, avec ou sans variations d'intensité, ou de façon discontinue avec des périodes d'obscurité entre les périodes d'éclairage, dans la mesure où il y a un effet sur le métabolisme des cellules exposées à ladite illumination. La périodicité de la variation de l'intensité ou de discontinuités peut être régulière au cours du temps, ou peut être modifié avec les différentes phases de la croissance cellulaire. La mixotrophie à dominante hétérotrophe permet de produire des molécules d'intérêt en couplant à la fois les avantages de l'autotrophie et de l'hétérotrophie. Sous ces conditions, comme en hétérotrophie, le substrat organique nourrit les microalgues pour produire de grandes quantités de biomasse, mais le chloroplaste et les autres organites capteurs d'énergie lumineuse de la cellule sont activés. La lumière agit comme signal pouvant induire une réponse du métabolisme, par exemple la synthèse de pigments.

Le terme "de caroténoïde" regroupe les carotènes (a, β, ε, γ, δ ou ζ-carotène, lycopène et phytoène) et les xanthophylles (astaxanthine, anthéraxanthine, citranaxanthine, cryptoxanthine, canthaxanthine, diadinoxanthine, diatoxanthine, flavoxanthine, fucoxanthine, lutéine, néoxanthine, rhodoxanthine, rubixanthine, siphonaxanthine, violaxanthine, zéaxanthine).

On entend avantageusement par "biomasse" selon l'invention, un ensemble de cellules de Thraustochytrides produites par la culture desdits protistes, et présentant les teneurs en protéines, en acides gras, et éventuellement en caroténoïdes, décrites dans le présent texte, cellules qui peuvent avoir conservé ou non leur intégrité physique.

On comprend donc que ladite biomasse peut comprendre une quantité de cellules de Thraustochytrides dégradées allant de 0% à 100%. Par "dégradé", on entend que lesdites cellules de Thraustochytrides peuvent avoir vu leur structure et/ou leur composition modifiées. Par exemple, elles peuvent avoir subi une étape de séchage ou alors une étape de récolte de leur huile, l'important étant que la biomasse comprenant ces cellules présente les teneurs en protéines, en acides gras, et éventuellement en caroténoïdes, décrites dans le présent texte.

Selon un mode préféré de réalisation de l'invention, la biomasse n'a pas subi de traitements qui modifient sa composition en acides aminés au cours ou après récolte, c'est à dire que les traitements que peut subir cette biomasse après récolte ne viennent pas en altérer la composition en acides aminés. En particulier, la biomasse n'a pas subi d'étape d'enrichissement en protéines et en acides aminés. Ces derniers proviennent de la croissance de cellules dans leur milieu de culture. C'est une biomasse dite « sans protéines ajoutées ».

Selon un mode plus préféré de l'invention, la biomasse n'a pas subi de traitements qui modifient sa composition en acides aminés et en matière grasse, et le cas échéant en caroténoïdes, c'est à dire que les traitements que subit cette biomasse après récolte ne viennent pas en altérer la composition en acides aminés et en matières grasses, et éventuellement en caroténoïdes. En particulier, la composition relative des acides aminés par rapport à la matière grasse reste substantiellement constante. Les acides gras, comme les protéines, proviennent de la seule culture des cellules dans leur milieu de culture. Sans enrichissement en acides gras, la biomasse est dite également « sans acides gras ajoutés ».

Il a été observé dans certains cas que les Thraustochytrides non dégradés dans la biomasse selon l'invention peuvent présenter de meilleures propriétés de conservation et de digestibilité que des Thraustochytrides dégradés. Une des formes préférées de l'invention est une biomasse comprenant une quantité substantiellement majoritaire de Thraustochytrides qui ne sont pas dégradés.

Par "dégradé", on entend selon l'invention des Thraustochytrides dont l'intégrité structurelle et/ou chimique a pu être altérée comme par exemple des Thraustochytrides lysés, résultant par exemple d'un procédé d'homogénéisation.

Il n'en demeure pas moins évident qu'une fois produite, ladite biomasse pourra être utilisée brute, séchée ou non, ou alors subir tout traitement nécessaire à son utilisation, notamment homogénéisée.

Selon l'invention, ladite biomasse peut présenter, en poids par rapport au poids de la matière sèche, un taux d'humidité de 1% à 95%.

Préférentiellement selon un mode de réalisation de l'invention, ladite biomasse peut présenter, en poids par rapport au poids de la matière sèche, un taux d'humidité de 70% à 90%, préférentiellement 80% à 85%.

Préférentiellement encore selon un deuxième mode de réalisation de l'invention, ladite biomasse peut présenter, en poids par rapport au poids de la matière sèche, un taux d'humidité de 1% à 10%, préférentiellement de 2% à 7%.

Selon l'invention, lesdits Thraustochytrides peuvent être de l'ordre des Thraustochytriales, préférentiellement de la sous-classe des Thraustochytriaceae, encore plus préférentiellement d'un genre pouvant être choisi dans le groupe comprenant les genres *Aurantiochytrium, Aplanochytrium, Botryochytrium, Japonochytrium, Oblongichytrium, Parietichytrium, Schizochytrium, Sicyoidochytrium, Thraustochytrium* et *Ulkenia.*

Les Thraustochytrides sont des microorganismes non génétiquement modifiés ou aussi des microorganismes génétiquement modifiés. Dans l'éventualité où des Thraustochytrides génétiquement modifiés seraient employés, ils ne contiennent pas de gènes codant pour une ou plusieurs enzymes qui permettent de dégrader ou digérer la biomasse en vue de son utilisation comme aliment.

Avantageusement, lesdits Thraustochytrides peuvent être choisis parmi les espèces *Aplanochytrium kerguelense* ; *Aplanochytrium minuta* ; *Aplanochytrium stocchinoi* ; *Aplanochytrium sp.* PR24-1 ; *Aurantiochytrium limacinum* ; *Aurantiochytrium limacinum* AB022107 ; *Aurantiochytrium limacinum* HM042909 ; *Aurantiochytrium limacinum* JN986842 ; *Aurantiochytrium limacinum* SL1101 JN986842 ; *Aurantiochytrium mangrovei* ; *Aurantiochytrium mangrovei* DQ323157 ; *Aurantiochytrium mangrovei* DQ356659 ; *Aurantiochytrium mangrovei* DQ367049 ; *Aurantiochytrium mangrovei* CCAP 4062/2 ; *Aurantiochytrium mangrovei* CCAP 4062/3 ; *Aurantiochytrium mangrovei* CCAP 4062/4 ; *Aurantiochytrium mangrovei* CCAP 4062/5 ; *Aurantiochytrium mangrovei* CCAP 4062/6 ; *Aurantiochytrium mangrovei* CCAP 4062/1 ; *Aurantiochytrium sp.* AB052555 ; *Aurantiochytrium sp.* AB073308 ; *Aurantiochytrium sp.* ATCC PRA276 DQ836628 ; *Aurantiochytrium sp.* BL10 FJ821477 ; *Aurantiochytrium sp.* LY 2012 PKU Mn5 JX847361 ; *Aurantiochytrium sp.* LY2012 JX847370 ; *Aurantiochytrium sp.* N1-27 ; *Aurantiochytrium sp.* SD116 ; *Aurantiochytrium sp.* SEK209 AB290574 ; *Aurantiochytrium sp.* SEK217 AB290572 ; *Aurantiochytrium sp.* SEK 218 AB290573 ; *Aurantiochytrium sp. 18W-13a* ; *Botryochytrium radiatum ; Botryochytrium radiatum* Raghukumar 16 ; *Botryochytrium radiatum* SEK353 ; *Botryochytrium sp.* ; *Botryochytrium sp.* BUTRBC 143 ; *Botryochytrium sp.* Raghukumar 29 ; *Oblongichytrium minutum ; Oblongichytrium multirudimentalis ; Oblongichytrium sp. ; Oblongichytrium sp.* SEK347 *; Parieticytrium sarkarianum ; Parieticytrium sarkarianum* SEK351 *; Parieticytrium sarkarianum* SEK364 *; Parieticytrium sp. ; Parieticytrium sp.* F3-1 *; Parieticytrium sp.* H1-14 *; Parieticytrium sp.* NBRC102984 ; *Phytophthora infestans* ; *Schizochytrium aggregatum* DQ323159 ; *Schizochytrium aggregatum* DQ356661 ; *Schizochytrium aggregatum ; Schizochytrium limacinum* ; *Schizochytrium limacinum* OUC166 HM042907 ; *Schizochytrium mangrovei* ; *Schizochytrium mangrovei* FB1 ; *Schizochytrium mangrovei* FB3 ; *Schizochytrium mangrovei* FB5 ; *Schizochytrium minutum* ; *Schizochytrium sp.* ATCC20888 DQ367050 ; *Schizochytrium sp.* KGS2 KC297137 ; *Schizochytrium sp.* SKA10 JQ248009 ; *Schizochytrium sp.* ATCC 20111 ; *Schizochytrium sp.* ATCC 20888 ; *Schizochytrium sp.* ATCC 20111 DQ323158* ; *Schizochytrium sp.* ATCC 20888 DQ356660 ; *Schizochytrium sp.* ATCC 20889 ; *Schizochytrium sp.* ATCC 26185 ; *Schizochytrium sp.* BR2.1.2 ; *Schizochytrium sp.* BUCAAA 032 ; *Schizochytrium sp.* BUCAAA 093 ; *Schizochytrium sp.* BUCACD 152 ; *Schizochytrium sp.* BUCARA 021 ; *Schizochytrium sp.* BUCHAO 113 ; *Schizochytrium sp.* BURABQ 133 ; *Schizochytrium sp.* BURARM 801 ; *Schizochytrium sp.* BURARM 802 ; *Schizochytrium sp.* CCAP 4087/3 ; *Schizochytrium sp.* CCAP 4087/1 ; *Schizochytrium sp.* CCAP 4087/4 ; *Schizochytrium sp.* CCAP 4087/5 ;*Schizochytrium sp.* FJU-512 ; *Schizochytrium sp.* KH105 ; *Schizochytrium sp.* KK17-3 ; *Schizochytrium sp.* KR-5 ; *Schizochytrium sp.* PJ10.4 ; *Schizochytrium sp.* SEK 210 ; *Schizochytrium sp.* SEK 345 ; *Schizochytrium sp.* SEK 346 ; *Schizochytrium sp.* SR21 ; *Schizochytrium sp.* TIO01 ; *Sicyoidochytrium minutum* SEK354 ; *Sicyoidochytrium minutum* NBRC 102975*Sicyoidochytrium minutum* NBRC 102979 ; *Thraustochytriidae sp.* BURABG162 DQ100295 ; *Thraustochytriidae sp.* CG9 ; *Thraustochytriidae sp.* LY2012 JX847378 ; *Thraustochytriidae sp.* MBIC11093 AB183664 ; *Thraustochytriidae sp.* NIOS1 AY705769 ; *Thraustochytriidae sp.* #32 DQ323161 ; *Thraustochytriidae sp.* #32 DQ356663 ; *Thraustochytriidae sp.* RT49 DQ323167 ; *Thraustochytriidae sp.* RT49 DQ356669 ; *Thraustochytriidae sp.* RT49 ; *Thraustochytriidae sp.* Thel2 DQ323162 ; *Thraustochytriidae* sp. Thel2 ; *Thraustochytrium aggregatum ; Thraustochytrium aggregatum* DQ356662 ; *Thraustochytrium aureum* ; *Thraustochytrium aureum* DQ356666 ; *Thraustochytrium gaertnerium* ; *Thraustochytrium kinnei* ; *Thraustochytrium kinnei* DQ323165 ; *Thraustochytrium motivum ; Thraustochytrium multirudimentale* ; *Thraustochytrium pachydermum* ; *Thraustochytrium roseum ; Thraustochytrium sp.* 13A4.1 ; *Thraustochytrium* sp. ATCC 26185 ; *Thraustochytrium sp.* BL13 ; *Thraustochytrium sp.* BL14 ; *Thraustochytrium sp.* BL2 ; *Thraustochytrium sp.* BL3 ; *Thraustochytrium sp.* BL4 ; *Thraustochytrium sp.* BL5 ; *Thraustochytrium sp.* BL6 ; *Thraustochytrium sp.* BL7 ; *Thraustochytrium sp.* BL8 ; *Thraustochytrium sp.* BL9 ; *Thraustochytrium sp.* BP3.2.2 ; *Thraustochytrium sp.* BP3.3.3 ; *Thraustochytrium sp.* caudivorum ; *Thraustochytrium sp.* CHN-1 ; *Thraustochytrium sp.* FJN-10 ; *Thraustochytrium sp.* HK1 ; *Thraustochytrium sp.* HK10 ; *Thraustochytrium sp.* HK5 ; *Thraustochytrium sp.* HK8 ; *Thraustochytrium sp.* HK8a ; *Thraustochytrium sp.* KK17-3 ; *Thraustochytrium sp.* KL1 ; *Thraustochytrium sp.* KL2 ; *Thraustochytrium sp.* KL2a ; *Thraustochytrium sp.* ONC-T18 ; *Thraustochytrium sp.* PJA10.2 *; Thraustochytrium sp.* TR1.4 ; *Thraustochytrium sp.* TRR2 ; *Thraustochytrium striatum* ; *Thraustochytrium striatum* ATCC24473 ; *Thraustochytrium striatum* DQ323163 ; *Thraustochytrium striatum* DQ356665 ; *Thraustochytrium visurgense* ; *Ulkenia amoeboidea* SEK 214 ; *Ulkenia profunda* ; *Ulkenia profunda* BUTRBG 111 ; *Ulkenia sp* ; *Ulkenia sp.* ATCC 28207 ; *Ulkenia visurgensis* ; *Ulkenia visurgensis* BURAAA 141 ; *Ulkenia visurgensis* ATCC 28208.

Préférentiellement selon l'invention les Thraustochytrides peuvent être choisis parmi les genres *Aurantiochytrium* et *Schyzochitrium*, préférentiellement parmi les espèces
*Aurantiochytrium mangrovei* CCAP 4062/2 déposée le 20 mai 2014 à la CCAP,
*Aurantiochytrium mangrovei* CCAP 4062/3 déposée le 20 mai 2014 à la CCAP,
*Aurantiochytrium mangrovei* CCAP 4062/4 déposée le 20 mai 2014 à la CCAP,
*Aurantiochytrium mangrovei* CCAP 4062/5 déposée le 20 mai 2014 à la CCAP,
*Aurantiochytrium mangrovei* CCAP 4062/6 déposée le 20 mai 2014 à la CCAP,
*Aurantiochytrium* CCAP 4062/1 déposée le 21 juin 2013 à la CCAP,
*Schizochytrium sp.* CCAP 4087/3 déposée le 20 mai 2014 à la CCAP,
*Schizochytrium sp.* CCAP 4087/1 déposée le 28 février 2012 à la CCAP,
*Schizochytrium sp.* CCAP 4087/4 déposée le 20 mai 2014 à la CCAP et
*Schizochytrium sp.* CCAP 4087/5 déposée le 20 mai 2014 à la CCAP.

Tous les dépôts ont été faits à la CCAP (CULTURE COLLECTION OF ALGAE AND PROTOZOA (CCAP), SAMS Research Services Ltd., Scottish Marine Institute, OBAN, Argyl PA37 1QA United Kingdom) selon les dispositions du traité de Budapest.

Selon des modes de réalisation préférés de l'invention, la biomasse peut avoir une teneur en protéines, en poids par rapport au poids de la matière sèche, d'au moins 40%, très préférentiellement de 35% à 55%. Selon des modes de réalisation préférés de l'invention la biomasse peut avoir une teneur en protéines, en poids par rapport au poids de la matière sèche comprise entre 40% et 65%.

Selon des modes de réalisation préférés de l'invention, la biomasse peut avoir une teneur en matière grasse, en poids par rapport au poids de la matière sèche, d'au moins 20%, préférentiellement d'au moins 30%, dont 25% à 60%, de préférence, 40% à 60% de PUFAs. En particulier, le rapport DHA : protéines (en poids par rapport au poids de la matière sèche) peut être compris entre 1 : 1,5 et 1 : 9, de préférence entre 1 : 2 et 1 : 7, plus préférentiellement entre 1 : 5 et 1 : 6.

Selon certains des modes de réalisation préférés de l'invention, la biomasse peut avoir éventuellement une teneur en caroténoïdes, comprise entre 5 et 250 ppm par rapport au poids de la matière sèche, de préférence entre 150 et 200 ppm. La teneur en caroténoïdes peut dépendre des conditions d'éclairage mais aussi du milieu de culture et de la conduite du procédé. Par conséquent, une même souche de micro-algue peut produire des quantités de pigments différentes en fonction des conditions de culture.

Selon des modes de réalisation préférés de l'invention, ladite biomasse susceptible d'être obtenue par le procédé selon l'invention peut être une biomasse :
- de Thraustochytrides pouvant être choisis parmi les genres *Aurantiochytrium* et *Schizochytrium,* préférentiellement parmi les espèces *Aurantiochytrium mangrovei* CCAP 4062/2 ; *Aurantiochytrium mangrovei* CCAP 4062/3 ; *Aurantiochytrium mangrovei* CCAP 4062/4 ; *Aurantiochytrium mangrovei* CCAP 4062/5 ; *Aurantiochytrium mangrovei* CCAP 4062/6 ; *Aurantiochytrium mangrovei* CCAP 4062/1 ; *Schizochytrium sp.* CCAP 4087/3 ; *Schizochytrium sp.* CCAP 4087/1 ; *Schizochytrium sp.* CCAP 4087/4 ; *Schizochytrium sp.* CCAP 4087/5 ; et pouvant présenter :
- une teneur en protéines, en poids par rapport au poids de la matière sèche, d'au moins 35%, de préférence d'au moins 40%, très préférentiellement de 35% à 55%, ou 45% à 55%;
- une teneur en matière grasse, en poids par rapport au poids de la matière sèche, d'au moins 20%, préférentiellement d'au moins 30%, dont 25 à 60%, de préférence, 40 à 60% de PUFAs;
- éventuellement une teneur en caroténoïdes, comprise entre 5 et 250 ppm par rapport au poids de la matière sèche, de préférence entre 150 et 200 ppm ; et
- un taux d'humidité avant séchage compris entre 70% et 90%, préférentiellement entre 80% et 85% ou un taux d'humidité après séchage compris entre 1% et 10%, préférentiellement entre 2% et 7%.

Les teneurs et types de caroténoïdes obtenus peuvent être contrôlés par le choix de la longueur d'onde de la lumière incidente, ainsi que par les conditions d'illumination. Les caroténoïdes tels que la lutéine, la fucoxanthine, l'astaxanthine, la zéaxanthine, la canthaxanthine, l'échinénone, le bêta-carotène et la phoenicoxanthine peuvent être produits selon des modes de réalisation de l'invention. Pour induire la production des caroténoïdes choisis parmi l'astaxanthine, la canthaxanthine, le bêta-carotène et la phoenicoxanthine, la culture est exposée à une lumière bleue (environ 435-475 mn, de préférence 455 nm). Selon un mode de réalisation, les caroténoïdes peuvent avoir une teneur par rapport à la matière grasse en astaxanthine entre 5% et 30%, une teneur en phoenicoxanthine entre 15% et 30% et une teneur en bêta-carotènes entre 0% et 30%.

L'invention a pour objet un procédé de production d'une biomasse telle que décrite précédemment qui comprend :
a. une première étape de culture en hétérotrophie ou en mixotrophie des Thraustochytrides dans un milieu de culture défini comprenant une source de carbone, une source d'azote, une source de phosphore et des sels, à une température comprise entre environ 24°C et 35°C et dans des conditions pouvant favoriser la production de protéines à une teneur d'au moins 40% de protéines en poids par rapport au poids de la matière sèche
   l'étape a) étant divisée en deux sous-étapes, une première sous-étape a1) de croissance dans le milieu de culture approprié jusqu'à obtenir des teneurs en source de carbone dans le milieu inférieure à 20 g/L, suivie d'une deuxième sous-étape a2) de production dans laquelle on ajoute au milieu de culture, simultanément ou successivement, une ou plusieurs solution(s) d'enrichissement en source de carbone, en source d'azote et en source de phosphore , la teneur en source de carbone est maintenue entre 0 et 50 g/L, la teneur en source d'azote est maintenue entre 0,5 et 5 g/L, et la teneur en source de phosphore est maintenue entre 0,5 et 5 g/L ;
b. une deuxième étape de culture en hétérotrophie ou en mixotrophie à une température entre environ 18°C et 25°C et inférieure à celle de l'étape a) dans des conditions pouvant favoriser l'accumulation de matière grasse jusqu'à au moins 20% en poids par rapport au poids de la matière sèche, de préférence au moins 30%, et pouvant favoriser l'accumulation des acides gras polyinsaturés (PUFAs), de préférence du DHA, et/ou de l'EPA, et/ou de l'ARA, au sein de la matière grasse jusqu'à au moins 25%, de préférence au moins 35% de la matière grasse, et éventuellement favorisant la production des caroténoïdes, jusqu'à l'obtention d'une densité de culture d'au moins 40 g/L en matière sèche, préférentiellement au moins 60 g/L, plus préférentiellement au moins 80 g/L ;
c. une troisième étape de récupération de la biomasse obtenue à la deuxième étape par séparation de ladite biomasse du milieu de culture (la récolte) ; et, le cas échéant
d. une quatrième étape de séchage de la biomasse récupérée à la troisième étape.

De manière préférentielle, l'étape a) de culture des Thraustochytrides peut être mise en œuvre dans un milieu de culture et dans des conditions appropriées pour favoriser la production de protéines.

De manière générale, ledit milieu de culture est un milieu de culture chimiquement défini qui comprend une source de carbone, une source d'azote, une source de phosphore et des sels connu de l'homme de métier pour la culture des Thraustochytrides, en l'adaptant pour les cellules qui ne se trouvent pas en carence d'azote durant l'étape a). On peut citer en tant qu'exemple les milieux à base de sels marins complémentés avec une source de carbone. On peut citer par exemple le milieu de type Verduyn modifié (sels marins 15 g/L, (NH₄)₂SO₄ 3g/L , KH₂PO₄ 1 g/L, MgSO₄ 7H₂O, 0,5 g/L, Na₂EDTA 24 mg/L, ZnSO₄·7H₂O 3 mg/L, MnCl₂·2H₂O 3 mg/L, Na₂MoO₄.2H2O 0,04 mg/L, FeSO₄·7H₂O 10mg/L, pantothénate 3,2 mg/L, hydrochlorure de thiamine 9,5 mg/L, vitamine B12 0,15 mg/L). On peut également citer des milieux de culture de type, ATCC790 MB2216 ou F/2.

Par "milieu de culture chimiquement défini", on entend selon l'invention un milieu de culture dans lequel la teneur de chaque élément est connue. Précisément, l'invention vise un milieu pouvant ne pas comporter de matières organiques riches ou complexes. Par matières organiques riches ou complexes, on entend des matières organiques non purifiées, se présentant sous la forme de mélanges pour lesquels la composition exacte et les concentrations des divers composants du mélange ne sont pas connues avec exactitude, pas maitrisées, et peuvent présenter une variabilité significative d'un lot à un autre. Comme exemple de matière organique riche ou complexe, on peut citer les extraits de levure ou les peptones qui sont des produits d'une réaction d'hydrolyse de protéines ou encore les matières minérales riches comme par exemple les sels minéraux marins ou autres agents de croissance complexes, n'ayant pas de concentration fixe de chacun de leurs composants.

Selon l'invention, ledit milieu défini peut comprendre des sels choisis parmi les sels de calcium, de cobalt, de manganèse, de magnésium, de zinc, de nickel, de cuivre, de potassium, de fer, de sodium, et leurs mélanges.

Avantageusement, lesdits sels peuvent être choisis parmi le chlorure de calcium, le chlorure de cobalt, le chlorure de manganèse, le sulfate de magnésium, le sulfate de zinc, le sulfate de nickel, le sulfate de cuivre, le sulfate de potassium, le sulfate ferreux, le sulfate de potassium, le molybdate de sodium, le sélénite de sodium, le chlorure de sodium et leurs mélanges.

Selon un mode de réalisation de l'invention et selon les souches employées, le milieu peut également comprendre du chlorure de sodium (NaCl), notamment pour certaines souches d'origine marine.

Selon ce mode de réalisation, on peut citer à titre d'exemple de souches marines pouvant admettre un milieu de culture pouvant comprendre du chlorure de sodium, les souches de *Schizochytrium sp.,* en particulier *Schizochytrium sp.* CCAP 4062/3 et CCAP4087/4.

Selon un autre mode de réalisation de l'invention et selon les souches employées, le milieu peut ne pas comprendre du chlorure de sodium (NaCl), à tout le moins comprendre une quantité de chlorure de sodium très faible, ayant moins de 3,5 g/L, de préférence moins de 1 g/L, plus préférentiellement moins de 10 mg/L d'ions de sodium et moins de 1 g/L, de préférence moins de 500 mg/L, plus préférentiellement 200 mg/L d'ions de chlorure.

Selon ce mode de réalisation, on peut citer à titre d'exemple de souches pouvant admettre un milieu de culture pouvant ne pas comprendre du chlorure de sodium (NaCl), à tout le moins comprendre une quantité de chlorure de sodium très faible, les souches *d'Aurantiochytrium mangrovei,* en particulier les souches *Aurantiochytrium mangrovei* CCAP 4062/4 et CCAP 4062/1.

Selon un mode de réalisation de l'invention, la source de carbone dudit milieu défini peut être un (ou des) glucide(s), un (ou des) acétate(s), un ou des alcools, une ou des molécules complexes, ou tout mélange, en toute proportion d'au moins deux de ces sources.

Par tout mélange, en toute proportion d'au moins deux de ces sources, on entend au moins l'un des mélanges suivants, en toute proportion : un (ou des) glucide(s) et un (ou des) acétate(s), un (ou des) glucide(s) et un (ou des) alcool(s), un (ou des) glucide(s) et une (ou des) molécule(s) complexe(s), un (ou des) acétate(s) et un (ou des) alcool(s), un (ou des) acétate(s) et une (ou des) molécule(s) complexe(s) et un (ou des) alcool(s) et une (ou des) molécule(s) complexe(s), un (ou des) glucide(s) et un (ou des) acétate(s) et un (ou des) alcool(s), un (ou des) glucide(s) et un (ou des) acétate(s) et une (ou des) molécule(s) complexe(s), un (ou des) glucide(s) et un (ou des) acétate(s) et un (ou des) alcool(s) et une (ou des) molécule(s) complexe(s).

Selon l'invention, ladite source d'azote dudit milieu défini peut-être choisie parmi un (ou des) sel(s) de nitrate, un (ou des) sel(s) de glutamate, un (ou des) sel(s) d'ammonium, l'urée, l'ammoniaque ou tout mélange, en toute proportion d'au moins deux de ces sources.

Par tout mélange, en toute proportion d'au moins deux de ces sources, on entend au moins l'un des mélanges suivants, en toute proportion : un (ou des) sel(s) de nitrate et un (ou des) sel(s) de glutamate et un (ou des) sel(s) d'ammonium et de l'urée et de l'ammoniaque ; un (ou des) sel(s) de nitrate et un (ou des) sel(s) de glutamate et un (ou des) sel(s) d'ammonium et de l'urée ; un (ou des) sel(s) de nitrate et un (ou des) sel(s) de glutamate et un (ou des) sel(s) d'ammonium et de l'ammoniaque ; un (ou des) sel(s) de nitrate et un (ou des) sel(s) de glutamate et de l'urée et de l'ammoniaque ; un (ou des) sel(s) de nitrate et un (ou des) sel(s) d'ammonium et de l'urée et de l'ammoniaque ; un (ou des) sel(s) de nitrate et un (ou des) sel(s) de glutamate et un (ou des) sel(s) d'ammonium ; un (ou des) sel(s) de nitrate et un (ou des) sel(s) de glutamate et de l'urée ; un (ou des) sel(s) de nitrate et un (ou des) sel(s) de glutamate et de l'ammoniaque ; un (ou des) sel(s) de nitrate et un (ou des) sel(s) d'ammonium et de l'urée ; un (ou des) sel(s) de nitrate et un (ou des) sel(s) d'ammonium et de l'ammoniaque ; un (ou des) sel(s) de nitrate et de l'urée et de l'ammoniaque ; un (ou des) sel(s) de glutamate et un (ou des) sel(s) d'ammonium et de l'urée et de l'ammoniaque ; un (ou des) sel(s) de glutamate et un (ou des) sel(s) d'ammonium et de l'urée ; un (ou des) sel(s) de glutamate et un (ou des) sel(s) d'ammonium et de l'ammoniaque ; un (ou des) sel(s) de glutamate et de l'urée et de l'ammoniaque ; un (ou des) sel(s) d'ammonium et de l'urée et de l'ammoniaque ; un (ou des) sel(s) de nitrate et un (ou des) sel(s) de glutamate ; un (ou des) sel(s) de nitrate et un (ou des) sel(s) d'ammonium ; un (ou des) sel(s) de nitrate et de l'urée ;
Selon un mode de réalisation de l'invention, la source de phosphore dudit milieu défini peut être choisie parmi l'acide phosphorique, les sels de phosphate, avantageusement de l'hydrogénophosphate de sodium (Na₂HPO₄), ou du dihydrogénophosphate de sodium (NaH₂PO₄), ou du dihydrogénophosphate de potassium (KH₂PO₄), ou de l'hydrogénophosphate de potassium (K₂HPO₄), ou tout mélange, en toute proportion d'au moins deux de ces sources.

Selon un mode de réalisation de l'invention, ledit milieu de culture pourra comprendre du chlorure de magnésium, avantageusement sous forme de tetrahydrate (MgCl₂ 4H₂O) ; du chlorure de calcium, avantageusement sous forme de dihydrate (CaCl₂ 2H₂O) ; du chlorure de cobalt hexahydrate (CoCl₂ 6H₂O) ; du chlorure de manganèse (II) tetrahydrate (MnCl₂ 4H₂O) ; du sulfate de magnésium heptahydrate (MgSO4, 7H2O) ; du sulfate de zinc heptahydrate (ZnSO4, 7H2O) ; du sulfate de nickel hexahydrate (NiSO₄ 6H₂O) ; du sulfate de cuivre pentahydrate (CuSO₄ 5H₂O) ; du sulfate de potassium (K₂SO₄) ; du sulfate ferreux heptahydrate (FeSO₄, 7H₂O) ; de l'acide borique (H₃BO₃) ; de l'acide éthylène diamine tétraacétique sous forme disodique dihydrate (Na₂EDTA, 2H₂O) ; du molybdate de sodium dihydrate, (Na₂MoO₄, 2H₂O) ; du sélénite de sodium, (Na₂SeO₃) ; à titre de vitamine de la thiamine, de la cobalamine ou vitamine B12, du panthoténate ou vitamine B5 ; une source de carbone ; une source d'azote ; une source de phosphore.

Selon une forme préférée de l'invention, dans ledit milieu de culture le chlorure de magnésium peut être à une concentration comprise entre 0,008 et 0,012 g/L, avantageusement entre 0,009 et 0,011 g/L ; le chlorure de calcium peut être à une concentration comprise entre 0,40 et 0,70 g/L, avantageusement entre 0,50 et 0,60 g/L ; le chlorure de cobalt hexahydrate peut être à une concentration comprise entre 0,00008 et 0,00013 g/L, avantageusement entre 0,00009 et 0,00012 g/L ; le chlorure de manganèse (II) tetrahydrate peut être à une concentration comprise entre 0,008 et 0,013, avantageusement entre 0,009 et 0,012 g/L ; le sulfate de magnésium heptahydrate peut être à une concentration comprise entre 6 et 10 g/L, avantageusement entre 7 et 9 g/L ; le sulfate de zinc heptahydrate peut être à une concentration comprise entre 0,008 et 0,013, avantageusement 0,009 et 0,012 g/L ; le sulfate de nickel hexahydrate peut être à une concentration comprise entre 0,004 et 0,007 g/L, avantageusement 0,005 et 0,006 g/L ; le sulfate de cuivre pentahydrate peut être à une concentration comprise entre 0,005 et 0,009 g/L, avantageusement entre 0,006 et 0,008 g/L ; le sulfate de potassium peut être à une concentration comprise entre 0,5 et 3,5 g/L, avantageusement entre 1 et 3 g/L ; le sulfate ferreux heptahydrate peut être à une concentration comprise entre 0,03 et 0,05 g/L, avantageusement entre 0,035 et 0,045 g/L ; l'acide borique peut être à une concentration comprise entre 0,0155 et 0,0195 g/L, avantageusement entre 0,0165 et 0,0185 g/L ; l'acide éthylène diamine tétraacétique sous forme disodique dihydrate peut être à une concentration comprise entre 0,10 et 0,14 g/L, avantageusement entre 0,11 et 0,13 g/L ; le molybdate de sodium dihydrate peut être à une concentration comprise entre 0,00001 et 0,0003 g/L, avantageusement entre 0,00005 et 0,0002 g/L ; le sélénite de sodium peut être à une concentration comprise entre 0,00000015 et 0,000019 g/L, avantageusement entre 0,00000016 et 0,00000018 g/L ; la thiamine peut être à une concentration comprise entre 0,015 et 0,05 g/L, avantageusement entre 0,025 et 0,04 g/L ; la cobalamine ou vitamine B12 peut être à une concentration comprise entre 0,0004 et 0,00065 g/L, avantageusement entre 0,00045 et 0,00060 g/L ; le panthoténate ou vitamine B5 peut être à une concentration comprise entre 0,008 et 0,013, avantageusement entre 0,009 et 0,012 g/L ; la source de carbone peut être à une concentration comprise entre 45 et 65 g/L, avantageusement entre 50 et 60 g/L ; la source d'azote peut être à une concentration comprise entre 7 et 11 g/L, avantageusement entre 8 et 10 g/L ; la source de phosphore peut être à une concentration comprise entre 2 et 6 g/L, avantageusement entre 3 et 5 g/L.

Très préférentiellement selon l'invention, dans ledit milieu de culture le chlorure de magnésium est à une concentration de 0,0108 g/L, le chlorure de calcium est à une concentration de 0,55 g/L ; le chlorure de cobalt hexahydrate (CoCl₂ 6H₂O) est à une concentration de 0,000108 g/L ; le chlorure de manganèse (II) tetrahydrate est à une concentration de 0,0108 g/L ; le sulfate de magnésium heptahydrate est à une concentration de 8,01 g/L ; le sulfate de zinc heptahydrate est à une concentration de 0,0108 g/L ; le sulfate de nickel hexahydrate est à une concentration de 0,0056 g/L ; le sulfate de cuivre pentahydrate est à une concentration de 0,0072 g/L ; le sulfate de potassium est à une concentration de 2,09 g/L ; le sulfate ferreux heptahydrate est à une concentration de 0,04 g :l ; l'acide borique est à une concentration comprise entre 0,0155 et 0,0195 g/L de 0,0175g/L ; l'acide éthylène diamine tétraacétique sous forme disodique dihydrate est à une concentration de 0,12 g/L ; le molybdate de sodium dihydrate, est à une concentration de 0,000108 g/L ; le sélénite de sodium, est à une concentration de 0,000000173 g/L ; la thiamine est à une concentration de 0,032 g/L ; la cobalamine ou vitamine B12 est à une concentration de 0,00052 g/L ; le panthoténate ou vitamine B5 est à une concentration de 0,0108 g/L ; la source de carbone est à une concentration de 55 g/L ; la source d'azote est à une concentration de 9 g/L ; la source de phosphore est à une concentration de 4 g/L.

Selon l'invention, la première étape a) de culture du procédé peut être réalisée en mode discontinu dit "batch", en mode semi-continu dit "fed batch" ou en mode d'alimentation continu.

Selon un autre mode de réalisation, la sous-étape a1) de croissance est mise en œuvre jusqu'à l'obtention d'une densité de culture d'au moins 40 g/L, plus préférentiellement au moins 60 g/L, encore plus préférentiellement au moins 80 g/L.

La teneur en source d'azote non limitante dans l'étape a2) est avantageusement comprise entre 0,5 et 5 g/L, préférentiellement entre 0,5 et 2 g/L et la teneur en source de phosphore non limitante est avantageusement comprise entre 0,5 et 5 g/L, préférentiellement 0,5 et 2 g/L. La teneur en source de carbone recherchée pour cette étape a2) pourra être comprise entre 0 et 50 g/L, plus préférentiellement entre 0 et 10 g/L.

De manière préférentielle, la culture dont la première étape a) est divisée en deux sous-étapes a1) et a2) telles que définies ci-dessus est réalisée en mode semi-continu dit "fed batch".

De manière générale, les sous étapes a1) et a2) sont effectuées à une température entre 24 et 35°C, de préférence entre 25 et 30°C.

La deuxième étape du procédé b) commence en général à partir d'environ 24h après le début de la culture. Elle est en général effectuée à une température entre 18 et 25°C. Cette baisse en température oriente la production vers des acides gras polyinsaturés de longues chaines (PUFAs), notamment de DHA, ARA, et EPA, au lieu des protéines. Le profil d'acide(s) gras produit(s) dépend, généralement, de la souche mise en culture.

Pendant l'étape b) de manière générale, comme on fait pour l'étape a2), on peut ajouter au milieu de culture, simultanément ou successivement, une ou plusieurs solution(s) d'enrichissement en source de carbone, et/ou en source d'azote et/ou en source de phosphore, de manière à maintenir dans le milieu de culture une teneur en source de carbone comprise entre 5 et 200 g/L, préférentiellement entre 10 et 50 g/L, une teneur en source d'azote comprise entre 0,5 et 5 g/L, préférentiellement entre 0,5 et 2 g/L et une teneur en source de phosphore comprise entre 0,5 et 5 g/L, préférentiellement 0,5 et 2 g/L.

La culture est arrêtée, de manière générale, entre 50 et 70 heures après le début de la culture.

On peut éventuellement induire la production des caroténoïdes pendant la culture, de préférence, pendant l'étape b) mais on peut le faire pendant la durée de la culture aussi. Pour induire la production des caroténoïdes, des conditions mixotrophiques avec une lumière, de préférence, bleue à environ 435-475 nm, de préférence 455 nm, sont appliquées. La culture est ainsi en mode mixotrophe. Des caroténoïdes tels que l'astaxanthine, la canthaxanthine, des bêta-carotènes et la phoenicoxanthine, sont produits avec ces longueurs d'onde. La lumière est de manière générale appliquée pour au moins 18 heures, de préférence pour au moins 24 heures. Selon un mode de réalisation préféré de l'invention, la lumière est appliquée à la culture à partir de 24 à 50 heures, plus préférentiellement, à partir de 45 heures de culture. Cet éclairement se poursuit, de manière générale, de préférence, jusqu'à la fin de la culture. Il est aussi possible d'arrêter l'éclairement peu de temps avant la fin de la culture (par exemple, un quart d'heure, un demi -heure, trois quarts heures ou une heure avant la fin).

Selon certains modes de réalisation de l'invention la culture est exposée à la lumière, en général, pour au moins 18 heures, de préférence au moins 24 heures avant la fin de la culture. Selon un mode de réalisation de l'invention, la culture est éclairée dès le départ, c'est-à-dire pendant les étapes a) et b) de culture. Selon un mode de réalisation préféré de l'invention, la culture est éclairée à partir du moment où l'étape b) commence. Selon un mode de réalisation préféré de l'invention, la culture est éclairée pendant au moins les dernières 18 heures de culture, de préférence pendant au moins les dernières 24 heures de culture.

On peut utiliser la lumière blanche, mais les inventeurs ont constaté des teneurs en caroténoïdes plus élevées en utilisant la lumière bleue, à une longueur d'onde d'environ 435-475 nm, de préférence 455 nm. En général, la culture est exposée à la lumière à partir de 40 à 50 heures de culture (c'est-à-dire, à partir de 40 à 50 heures après le début de la de culture), de préférence, à partir de 45 heures de culture et jusqu'à la fin de la culture (entre 50 et 70 heures). Dans ces conditions, on obtient une teneur en caroténoïdes d'environ 150-250 ppm (ng/mg), de préférence 180 à 200 ppm (matière sèche). Selon un mode de réalisation, des caroténoïdes ont une teneur en astaxanthine entre 5 et 30%. Selon un mode de réalisation, des caroténoïdes ont une teneur en phoenicoxanthine entre 15 et 30%. Selon un mode de réalisation, des caroténoïdes ont une teneur en bêta-carotènes entre 0 et 30%.

De manière générale, les étapes a) et b) du procédé peuvent être effectuées en conditions hétérotrophiques et/ou en conditions mixotrophiques. Pour obtenir des caroténoïdes, dans l'étape b) la culture est effectuée en mixotrophie pour au moins 24 heures avant la fin de la culture.

Selon un mode de l'invention, par exemple quand on ne produit pas de caroténoïdes, la culture est menée entièrement en conditions d'hétérotrophie. En général, l'utilisation des conditions de mixotrophie lors de la culture a un effet positif sur la production des lipides, notamment des PUFAs, ainsi que sur la production des caroténoïdes, et permet d'augmenter le rendement en biomasse, en acides gras, notamment en PUFAs, en particulier en DHA et en caroténoïdes.

Des étapes a1), a2) et b) peuvent être effectuées indépendamment en conditions d'hétérotrophie ou en conditions de mixotrophie. Selon un mode de réalisation de l'invention, les étapes a1) et a2) sont menées en conditions d'hétérotrophie, puis l'étape b), entièrement ou en partie, en conditions de mixotrophie. Selon un mode de réalisation préféré de l'invention, l'étape a1) est menée en conditions d'hétérotrophie et l'étape a2) en conditions de mixotrophie.

Selon un autre mode de réalisation de l'invention, pendant l'étape b) la culture est exposée, en conditions de mixotrophie, à une lumière blanche. Selon un autre mode de réalisation de l'invention, pendant l'étape b) la culture est exposée, en conditions de mixotrophie, à une lumière blanche, jusqu'à 40 à 50, de préférence 45 heures de culture, puis la culture est exposée à une lumière bleue avec une longueur d'onde d'environ 435-475 nm, de préférence 455 nm pour au moins 24 heures.

Selon un mode de réalisation de l'invention, dans la mise en œuvre d'une culture en mode mixotrophe, l'éclairement est variable et/ou discontinu. Un éclairement sous formes de flashs est particulièrement favorable sur le développement des protistes et permet d'accroître la productivité de ceux-ci, notamment en ce qui concerne leur production de lipides et de caroténoïdes.

Par "éclairement discontinu", il faut entendre un éclairement ponctué par des périodes d'obscurité. Les périodes d'obscurité peuvent occuper plus d'un quart du temps, de préférence la moitié du temps ou plus, durant lequel les algues sont cultivées.

Selon un aspect préféré de l'invention, l'éclairement est discontinu et plus préférentiellement sous forme de flashs. Un flash, au sens de l'invention, est un éclairement lumineux de courte durée, c'est-à-dire de moins de 30 minutes. La durée peut être de moins de 15 minutes, de préférence de moins de 5 minutes ou plus préférentiellement encore de moins de 1 minute. Selon certains modes de réalisation de l'invention, la durée du flash peut être de moins d'une seconde. Par exemple, le durée du flash peut être 1/10 d'une seconde, ou 2/10 d'une seconde, ou 3/10 d'une seconde, ou 4/10 d'une seconde, ou 5/10 d'une seconde, ou 6/10 d'une seconde, ou 7/10 d'une seconde, ou 8/10 d'une seconde, ou 9/10 d'une seconde. L'éclairement lumineux, ou le flash, est généralement d'une durée supérieure à 15 secondes. Elle est généralement comprise entre 5 secondes et 10 minutes, de préférence entre 10 secondes et 2 minutes, plus préférentiellement entre 20 secondes et 1 minute. Ces durées de flash sont appropriées pour des apports de lumière de "basse fréquence".

Selon ce dernier mode de réalisation où le régime d'illumination (l'apport de la lumière) est de "basse fréquence", le nombre de flashs peut être compris entre environ 2 et 3600 par heure. Il peut être, par exemple, compris entre 100 et 3600 flashs par heure. Il peut être également compris entre 120 et 3000, ou entre 400 et 2500, ou entre 600 et 2000, ou entre 800 et 1500 flashs par heure. Il peut être également compris entre 2 et 200, préférentiellement entre 10 et 150, plus préférentiellement entre 15 et 100, et plus préférentiellement encore entre 20 et 50 par heure.

Selon un mode de réalisation, un flash peut avoir une durée entre 1/150000 seconde et 1/1000 seconde. Ces durées de flash sont appropriées pour des régimes d'illumination de "haute fréquence" ; c'est-à-dire avec des fréquences de flash de 150kHz à 1kHz respectivement.

Selon ce mode de réalisation où le régime d'illumination (l'apport de la lumière) est de "haute fréquence", les flashs peuvent avoir lieu entre 3,6 ×10⁵ et 5,4 × 10⁹ fois par heure. Dans ce cas, la variation de la lumière a une fréquence entre 1 kHz et 150 kHz, c'est-à-dire entre 1000 et 150 000 flashes par seconde. L'apport de lumière selon ce dernier mode de réalisation de l'invention est nommé "haute fréquence".

Le nombre de flashs par heure peut être choisi en fonction de l'intensité et de la durée des flashs (voir ci-dessous). En général, l'intensité de la lumière apportée sous forme de flashs peut être comprise entre 5 et 1000 µmol.m⁻². s⁻¹, de préférence entre 5 et 500 µmol. m⁻². s⁻¹, ou 50 et 400 µmol. m⁻². s⁻¹, et plus préférentiellement entre 150 et 300 µmol. m⁻². s⁻¹. 1 µmol. m⁻². s⁻¹ correspond à 1µE m⁻². s⁻¹ (Einstein), unité souvent utilisée dans la littérature.

Selon un autre mode de l'invention, l'éclairement peut être variable, ce qui signifie que l'éclairement n'est pas interrompu par des phases d'obscurité, mais que l'intensité lumineuse varie au cours du temps. Cette variation de l'intensité de lumière peut être régulière et peut être périodique ou cyclique. Selon l'invention, on peut aussi procéder à un apport lumineux alliant des phases d'éclairement continues et discontinues.

Selon un mode de réalisation préféré de l'invention, des conditions de mixotrophie à dominante hétérotrophe sont utilisées. Les thraustochytrides ne possèdent pas de chloropastes. Par conséquent, la majorité de l'énergie utilisée pour la croissance vient de la source de carbone du milieu de culture. Dans ce cas, la lumière induit certaines voies métaboliques dans la cellule mais ne permet pas de fixer le carbone par la photosynthèse.

Selon un mode de réalisation préféré, l'éclairage des cultures peut être assuré par un dispositif d'illumination interne au fermenteur. Ainsi, l'efficacité de l'illumination est meilleure par rapport à une configuration où la lumière pénètre par des hublots à partir de sources disposées à l'extérieur. Les dispositifs d'illumination peuvent être disposés sur l'ensemble tournant muni de pales ou sur des pièces tubulaires plongeant dans la masse à traiter, ou encore au fond ou au plafond de la cuve. Selon un mode de réalisation préféré de l'invention, le dispositif d'illumination peut être situé sur les contre-pales à l'intérieur du fermenteur. Un tel dispositif est décrit dans la demande de brevet français Ne°1353641. Le fermenteur peut être ainsi muni d'une pluralité de sources d'illumination portées par des contre-pales, ces dernières ayant pour fonction d'empêcher la formation d'un vortex au sein de la biomasse sous l'action de l'ensemble tournant de brassage. Ces sources d'illumination sont de préférence encapsulées, partiellement ou complètement dans au moins une partie de ces contre pales, dans une matière compatible avec la biomasse et en une épaisseur permettant de diffuser ladite lumière vers l'intérieur de la cuve.

Selon l'invention, la culture peut être réalisée par toute technique de culture connue, par exemple en fioles ou en réacteur, mais aussi en fermenteurs ou encore dans tout contenant apte à la croissance de protistes, particulièrement de Thraustochytrides, comme par exemple des bassins de type "raceway", à condition que ladite technique permette de mettre en œuvre les conditions de culture requises.

De manière préférentielle, la culture peut être réalisée en fermenteurs selon les méthodes connues de culture des protistes en fermenteurs.

Selon l'invention, la troisième étape c) du procédé permettant la récupération de ladite biomasse peut être réalisée dans des conditions appropriées pour obtenir une biomasse pouvant présenter le taux d'humidité recherché.

Ladite récupération des protistes peut être réalisée par toute technique permettant la récupération de la biomasse, notamment les méthodes de filtration, gravimétrique ou sous pression réduite, de centrifugation, de décantation, ou bien encore des méthodes de précipitation suivie d'une filtration gravimétrique.

L'invention a aussi pour objet une biomasse susceptible d'être obtenue par le procédé selon l'invention tel que décrit précédemment dans toutes ses variantes.

L'invention a encore pour objet l'utilisation d'une biomasse telle que décrite précédemment dans les domaines cosmétiques, pharmaceutiques, alimentaires (alimentation humaine ou animale).

On pourra distinguer dans l'alimentation animale, l'alimentation des animaux d'élevage, en particulier en élevage industriel et celle des animaux domestiques ou encore des animaux de compagnie ou des animaux dits de "loisir", comme les poissons d'aquarium ou les oiseaux de volière ou en cage.

Par "animal d'élevage" on entend notamment les animaux de pacage (notamment les bovins élevés pour la viande, le lait, le fromage et le cuir ; les ovins élevés pour la viande, la laine et le fromage ; les caprins), les porcins, les lapins, les volailles (les poulets, les poules, les dindes, les canards, les oies et autres), les équidés (poneys, chevaux, poulains), destinés à supporter des activités humaines (transport, loisirs) ou leur alimentation, les animaux aquatiques (par exemple les poissons, les crevettes, les huîtres et les moules). On pourra toutefois distinguer l'alimentation des poissons jusqu'au stade d'alevins, et celle des poissons élevés, y compris les aliments et compositions alimentaires qui leur sont destinées.

On les distinguera des animaux domestiques, animaux de compagnie ou animaux de loisir. Ils comprennent également des mammifères, ruminants ou non, des oiseaux ou des poissons. Ils comprennent en particulier les chiens et les chats.

L'invention a aussi pour objet un aliment, ou composition alimentaire, pour l'homme ou les animaux, pouvant comprendre une biomasse selon l'invention telle que décrite précédemment. On entend par "aliment" toute composition qui peut servir à la nourriture de l'homme ou des animaux, en particulier les animaux d'élevage.

Selon un mode de réalisation de l'invention, l'aliment peut être constitué de la biomasse, séchée ou non, transformée ou non, ou de la biomasse, séchée ou non, transformée ou non, mélangée à tout autre additif, véhicule ou support, utilisé dans le domaine de l'alimentation humaine ou animale. On peut citer comme exemple en tant qu'additifs les conservateurs alimentaires, les colorants, les exhausteurs de goût, les régulateurs du pH, les charges, les agents épaississants, texturants ou les agents liants. On peut également citer des vitamines, des probiotiques et des prébiotiques, ou encore des additifs pharmaceutiques comme par exemple des hormones de croissance, des antibiotiques.

Selon un mode de réalisation de l'invention, la biomasse, séchée ou non, transformée ou non, peut être utilisée elle-même en tant que véhicule ou support, additif, conservateur alimentaire, colorant, exhausteur de goût, charge, agent épaississant, texturant ou agent liant dans un produit destiné à l'alimentation humaine ou animale.

La présente invention concerne en particulier des aliments pour animaux et plus particulièrement pour les animaux d'élevage. Ces aliments peuvent se présenter habituellement sous la forme de mélanges simples, de farines, de granulés, de soupe ou de fourrages (fourrage frais, ensilages, foin...) dans lesquels peut être incorporée la biomasse selon l'invention. Ces aliments peuvent être réalisés par l'éleveur lui-même ou fabriqués chez un professionnel de l'alimentation animale. On entend par "aliment" tout ce qui peut servir à la nourriture des animaux.

Pour l'élevage intensif des animaux, les aliments peuvent comprendre, en plus de la biomasse algale, une base nutritionnelle et des additifs nutritionnels. L'essentiel de la ration alimentaire de l'animal peut être alors constituée par la "base nutritionnelle" et la biomasse algale. Cette base peut être constituée à titre d'exemple par un mélange de céréales, de protéines et de matières grasses d'origine animale et/ou végétale.

Les bases nutritionnelles pour animaux sont adaptées à l'alimentation de ces animaux et sont bien connues de l'homme du métier. Dans le cadre de la présente invention, ces bases nutritionnelles peuvent comprendre par exemple de la farine de poisson, des dérivés du maïs, du blé, des pois et du soja et autres graines. Ces bases nutritionnelles peuvent être adaptées aux besoins des différentes espèces animales auxquelles elles sont destinées. Ces bases nutritionnelles peuvent déjà contenir des additifs nutritionnels comme des vitamines, des sels minéraux et des acides aminés.

Les additifs utilisés en alimentation animale peuvent être ajoutés à la base nutritionnelle ou à la biomasse algale. Ces additifs peuvent être ajoutés pour améliorer certaines caractéristiques des aliments, par exemple pour en relever le goût, pour rendre plus digestes les matières premières des aliments pour animaux, conserver plus efficacement les aliments ou protéger les animaux. Ils sont fréquemment utilisés dans les élevages intensifs de grande envergure.

Les additifs pouvant être utilisés en alimentation animale se déclinent, notamment, dans les sous-catégories suivantes (source EFSA) :
- additifs technologiques : par exemple, conservateurs, antioxydants, émulsifiants, stabilisateurs, régulateurs d'acidité et additifs pour l'ensilage ;
- additifs sensoriels : par exemple, arômes, colorants,
- additifs nutritionnels : par exemple, vitamines, acides aminés, oligo-éléments ;
- additifs zootechniques : par exemple, améliorateurs de digestibilité, stabilisateurs de flore intestinale, tels que des probiotiques et prébiotiques ;
- coccidiostatiques et histomonostatiques (antiparasitaires).

Dans un mode de réalisation, l'invention concerne des aliments pour animaux d'élevage pouvant comprendre entre 1% et 60%, de préférence entre 1 et 20%, de façon tout à fait préférentielle entre 3% et 8% d'une biomasse séchée ou obtenue par le procédé selon l'invention.

Dans un autre mode de réalisation, l'invention concerne des aliments pour animaux d'élevage pouvant comprendre entre 1% et 40%, de préférence entre 5 et 1 0% d'une biomasse non séchée obtenue par le procédé de l'invention.

Selon un mode particulier de réalisation de l'invention, l'aliment peut être destiné aux animaux d'élevage, en particulier les bovins, les ovins, les porcins, les lapins, les volailles et les équidés.

Selon un autre mode particulier de l'invention, l'aliment peut être destiné aux animaux aquatiques, en particulier aux poissons, au moins jusqu'au stade alevins, voire y compris les poissons d'élevage. L'utilisation d'une biomasse riche en protéines et PUFAs et comprenant des pigments en fin de phase de croissance permet d'améliorer la qualité de la chair (grâce à la présence des PUFAs et des pigments).

Selon un autre mode particulier de réalisation de l'invention, l'aliment peut être destiné aux animaux domestiques, animaux de compagnie et/ou animaux de loisirs.

Enfin, selon un autre mode de réalisation de l'invention, la composition alimentaire peut être destinée à l'homme, en particulier, la composition peut être adaptée aux enfants, adolescents, adultes ou personnes âgées,

L'invention a aussi pour objet l'utilisation de la biomasse telle que décrite précédemment en thérapie, par exemple dans la prévention et le traitement de la malnutrition. Il est connu que dans le traitement de la malnutrition, la carence en protéine est la plus préjudiciable et coûteuse à traiter. La biomasse, selon un mode de réalisation de l'invention, peut constituer une solution complète de choix pour prévenir et/ou traiter la malnutrition, notamment des enfants et les séniors. Elle peut constituer une source protéique équilibrée et digestible (la carence en protéine est la plus préjudiciable et couteuse). Sa fraction protéique peut être associée à une source de lipides de haute qualité nutritionnelle nécessaires à un bon développement sain des enfants. La biomasse selon l'invention peut être également adaptée pour utilisation par des personnes âgées pour la prévention et/ou le traitement de la malnutrition, et des autres maladies liées au vieillissement. Dans ce cadre, l'apport en caroténoïdes de la biomasse selon l'invention peut être également très bénéfique pour la santé de ces populations, les personnes âgées étant susceptibles aux maladies telles que le DMLA.

La biomasse peut donc être incorporée dans un aliment complet (sous forme humide ou de poudre destinées à être réhydratées) un aliment de collation tels que des barres de céréales, des biscottes, des gâteaux secs ou non, des confiseries, des produits de cuisson tels que des crèmes, des poudres en mix, des produits laitiers, des céréales ou des boissons. La biomasse peut être présente dans des suppléments alimentaires, par exemple sous forme de poudre, pâte à tartiner ou sauces à ajouter à l'alimentation quotidienne.

### EXEMPLES

D'autres aspects et caractéristiques de l'invention pourront apparaitre à la lecture des exemples qui suivent.

### Exemple 1 : Procédé de culture à 25°C

Les cultures *d'Aurantiochytrium* CCAP4062/1 ont été réalisées dans des fermenteurs (bioréacteurs) de 1 à 2 L utiles avec automates dédiés et supervision par station informatique. Le système a été régulé en pH via l'ajout de base (NH₄OH,). La température de culture a été fixée à 25°C puis descendu à 18°C à 24h de culture. La pression d'oxygène dissous a été régulée dans le milieu tout au long de la culture, par la vitesse d'agitation (220 - 1200 t/min), le débit d'air (0,6 - 2 vvm), voire le débit d'oxygène (0 - 2 vvm). Les paramètres de régulation, intégrés dans l'automate de supervision, ont permis de maintenir une pO₂ constante comprise entre 5% et 30%. Le temps de culture a été de 72 heures.

La composition du milieu de culture utilisé pendant la culture est donnée dans le tableau:

| **Solution principale** | **Concentration g/L** |
|---|---|
| KCI | 0,36 |
| H₃BO₃ | 0,175 |
| M₈SO₄,7H₂O | 6,750 |
| CaCl₂,2H₂O | 0,55 |
| KNO₃ | 0,04667 |
| KH₂PO₄,7H₂O | 0,30940 |
| Na₂EDTA,2H₂O | 3,094.10⁻³ |
| ZnSO₄,7H₂O | 7,3.10⁻⁵ |
| CoCl₂,6H₂O | 1,6.10⁻⁵ |
| MnCl₂,4H₂O | 5,4.10⁻⁴ |
| Na₂MoO₄,2H₂O | 1,48.10⁻⁶ |
| Na₂SeO₃ | 1,73.10⁻⁶ |
| NiSO₄,6H₂O | 2,98.10⁻⁶ |
| CuSO₄,5H₂O | 9,8.10⁻⁶ |
| EDTA-Fe | 0,03 |
| **Carbone** | **g/L** |
| Glucose | 55 |
| **Azote** | **g/L** |
| (NH₄)₂SO₄ | 7 |

| **Ajout après autoclave** | |
|---|---|
| **Vitamines** | **g/L** |
| Thiamine | 8.10⁻³ |
| Vitamine B12 | 1,3.10⁻⁴ |
| Panthothénate | 2,7.10⁻³ |

| **Solution d'ajout 1** | **Concentration g/L** |
|---|---|
| K₂SO₄ | 31,9 |
| M₈SO₄,7H₂O | 25,8 |
| KH₂PO₄,7H₂O | 61,38 |
| FeSO₄,7H₂O | 0,61 |
| (NH₄)₂SO₄ * | 138,24 |
| MnCl₂ 4H₂O | 0,165 |
| ZnSO₄,7H₂O | 0,165 |
| CoCl₂,6H₂O | 1,6.10⁻³ |
| Na₂MoO₄,2H₂O | 1,6.10⁻³ |
| CuSO₄,5H₂O | 0,11 |
| NiSO₄,6H₂O | 8,6.10⁻² |
| Na₂EDTA,2H₂O | 1,81 |
| Thiamine | 0,49 |
| Vitamine B12 | 8.10⁻³ |
| Panthothénate | 0,1656 |

| **Solution d'ajout 2** | **Concentration g/L** |
|---|---|
| Glucose | 750 |
| KH₂PO₄ | 6,4 |
| (NH₄)2SO₄* | 34 |

Des ajouts de glucoses ont été faits sous forme des solutions d'enrichissement ayant un ratio molaire de carbone à phosphore (C :P) de 530 :1.

Des précultures ont été réalisées sur une table d'agitation (140 t/min) en enceinte à température contrôlée (26°C), une première de 24h puis de 70h.

### Suivi des cultures :

La concentration en biomasse totale a été suivie par mesure de la masse sèche (filtration sur filtre GF/F, Whatman, puis séchage en étuve, à 105°C, pendant 24 h minimum avant pesée). L'aspect de la biomasse est beige clair.

Les analyses en contenus lipidiques totaux et des acides gras ont été réalisées selon les méthodes classiquement décrites dans la littérature [Folch J, *et al.,* A simple method for the isolation and purification of total lipides from animal tissues. J Biol Chem. 1957 May ; 226(1) :497-509].

Résultats :

| Référence Essai | Temps culture | MS (g/L) | % MG/MS | % AA/MS | % DHA/MG |
|---|---|---|---|---|---|
| Essai A | 71h | 95 | 25,8 | 44,6 | 29 |
| Essai B | 71h | 105 | 27,9 | 45,2 | 32 |

### Exemple 2 : Procédé de culture avec étape a) à 30°C

Les cultures *d'Aurantiochytrium* CCAP4062/4 ont été réalisées dans des fermenteurs (bioréacteurs) de 1 à 2 L utiles avec automates dédiés et supervision par station informatique. Le système a été régulé à pH 6 via l'ajout de base (NH₄OH). La température de culture a été fixée à 30°C puis descendu à 18°C à 24h de culture. La pression d'oxygène dissous a été régulée dans le milieu tout au long de la culture, par la vitesse d'agitation (220 - 1200 t/min), le débit d'air (0,3 - 1 vvm), voire le débit d'oxygène (0 - 1 vvm). Les paramètres de régulation, intégrés dans l'automate de supervision, ont permis de maintenir une pO₂ constante comprise entre 5% et 30%. Le temps de culture a été compris entre 40 et 100 heures (essai A : 69 heures, essai B : 51 heures)

Le milieu de culture est le même que celui de l'exemple 1.

Des ajouts de glucose sous forme d'une solution d'enrichissement ayant un ratio molaire de carbone : azote : phosphore (CNP) de 190,5 : 7,3 : 1 ont été faits pour l'essai A. Pour l'essai B la solution d'enrichissement et avait un ratio molaire CNP de 95,3 : 3,2 : 1

Un chainage de préculture a été réalisé sur table d'agitation (140 t/min) en enceinte à température contrôlée (26°C), une première de 24h puis de 70h.

### Suivi des cultures :

La concentration en biomasse totale a été suivie par mesure de la masse sèche (filtration sur filtre GF/F, Whatman, puis séchage en étuve, à 105°C, pendant 24 h minimum avant pesée). L'aspect de la biomasse est beige clair.

Les analyses en contenus lipidiques totaux et des acides gras ont été réalisées selon les méthodes classiquement décrites dans la littérature [Folch J, *et al.,* A simple method for the isolation and purification of total lipides from animal tissues. J Biol Chem. 1957 May ; 226(1) :497-509].

| Référence Essai | Temps culture | MS (g/L) | % MG/MS | % AA | %DHA/MG |
|---|---|---|---|---|---|
| Essai A | 69h | 128 | 22 | 48 | 37 |
| Essai B | 51h | 130 | 24 | 40 | 33 |

### Exemple 3 : Procédé de culture avec éclairage en lumière bleue pendant les derniers 25 heures de l'étape b)

Les cultures *d'Aurantiochytrium* CCAP4062/4 ont été réalisées dans des fermenteurs (bioréacteurs) de 5 L utiles avec automates dédiés et supervision par station informatique. Le système a été régulé à pH 5 via l'ajout de base (NaOH). La température de culture a été fixée à 30°C puis descendu à 18°C à 24h de culture. Les paramètres de régulation, intégrés dans l'automate de supervision, ont permis de maintenir une pO₂ constante comprise entre 5% et 30%. Le temps de culture a été de 70 heures. La culture a été exposée à lumière bleue (longueur d'onde 455 nm) à partir de 45h de culture. Les sources de lumières sont en forme de LEDs (ou DELs en français, pour diode électroluminescente) ont étés montés sur deux contre-pales dans le bioréacteur.

Le milieu de culture est le même que celui de l'Exemple 1.

Des ajouts de glucose sous forme d'une solution d'enrichissement avec un ratio molaire CNP de 37 : 4 : 1 ont été faits.

Un chainage de préculture a été réalisé sur table d'agitation (140 t/min) en enceinte à température contrôlée (26°C), une première de 24h puis de 70h. Cela était suivi d'une préculture en fermenteur de 24h.

### Suivi des cultures :

La concentration en biomasse totale a été suivie par mesure de la masse sèche (filtration sur filtre GF/F, Whatman, puis séchage en étuve, à 105°C, pendant 24 h minimum avant pesée).

Les analyses en contenus lipidiques totaux ont été réalisées selon les méthodes classiquement décrites dans la littérature [Folch J, *et al.,* A simple method for the isolation and purification of total lipides from animal tissues. J Biol Chem. 1957 May ; 226(1) :497-509].

| Référence Essai | Temps culture | MS (g/L) | % MG/MS | % AA | % DHA/MG |
|---|---|---|---|---|---|
| Essai L.A | 70h | 130 | 23 | 51 | 30 |

L'aspect de la biomasse est jaune/orange. La différence de couleur par rapport les Exemples 1 et 2 qui ont donné une biomasse de couleur blanc/crème claire est due à la présence de caroténoïdes (principalement bêta-carotènes, cantaxanthine, phoenicoxanthine et astaxanthine) dans le flacon de gauche.

Caroténoïdes mesuré dans l'essai A : 199 ppm.

| | Astaxanthine | Phenicoxanthine | Canthaxanthine |
|---|---|---|---|
| ppm* | 32 | 50,9 | 116 |
| % de caroténoïdes totaux | 16% | 26% | 58% |

| | | | |
|---|---|---|---|
| * Ppm= ng/mg de MS. | | | |

## Revendications

1. Procédé de préparation d'une biomasse de Thraustochytrides dont une quantité substantiellement majoritaire n'est pas dégradée, comprenant des protéines et des acides gras, **caractérisée en ce qu'**elle comprend en poids par rapport au poids de la matière sèche, au moins 40% de protéines et au moins 20%, de matière grasse, la matière grasse ayant une teneur de 25 à 60% en acides gras polyinsaturés choisi parmi l'acide arachidonique (AA), l'acide docosahexaénoïque (DHA) et l'acide éicosapentaénoïque (EPA), sans protéines ni acides gras ajoutés , **caractérisé en ce qu'**il comprend :
a. une première étape de culture des Thraustochytrides réalisée en mode dit "batch", en mode dit "fed batch" ou en mode continu dans des conditions de hétérotrophie ou de mixotrophie dans un milieu de culture défini comprenant une source de carbone, une source d'azote, une source de phosphore et des sels, à une température comprise entre environ 24°C et 35°C et dans des conditions pour favoriser la production de protéines à une teneur d'au moins 40% de protéines en poids par rapport au poids de la matière sèche,
l'étape a) étant divisée en deux sous-étapes, une première sous-étape a1) de croissance dans le milieu de culture approprié jusqu'à obtenir des teneurs en source de carbone dans le milieu inférieure à 20 g/L, suivie d'une deuxième sous-étape a2) de production dans laquelle on ajoute au milieu de culture, simultanément ou successivement, une ou plusieurs solution(s) d'enrichissement en source de carbone, en source d'azote et en source de phosphore , la teneur en source de carbone est maintenue entre 0 et 50 g/L, la teneur en source d'azote est maintenue entre 0,5 et 5 g/L, et la teneur en source de phosphore est maintenue entre 0,5 et 5 g/L ;
b. une deuxième étape de culture dans des conditions de hétérotrophie ou de mixotrophie à une température entre environ 18°C et 25°C et inférieure à celle de l'étape a) dans des conditions pour favoriser l'accumulation de matière grasse jusqu'à au moins 20% en poids par rapport au poids de la matière sèche, et jusqu'à l'obtention d'une densité de culture d'au moins 40 g/L en matière sèche ;
c. une troisième étape de récupération de la biomasse obtenue à la deuxième étape par séparation de ladite biomasse du milieu de culture (la récolte), ; et, le cas échéant
d. une quatrième étape de séchage de la biomasse récupérée à la troisième étape.

2. Procédé selon la revendication 1, caractérisé en ce la culture est éclairée, avec une lumière de longueur d'onde entre 435 et 475 nm.

3. Procédé selon la revendication 2, **caractérisé en ce que** la culture est éclairée pendant au moins les dernières 18 heures de culture.

4. Procédé selon l'une des revendications 1 à 3, **caractérisée en ce que** la biomasse comprend au moins 45% de protéines.

5. Procédé selon l'une des revendications 1 à 4, **caractérisée en ce qu'**elle comprend au moins 30% de matière grasse.

6. Procédé selon l'une quelconque des revendications 1 à 5, **caractérisée en ce que** lesdits Thraustochytrides sont d'un genre choisi dans le groupe comprenant *Aurantiochytrium, Aplanochytrium, Botryochytrium, Japonochytrium, Oblongichytrium, Parietichytrium, Schizochytrium, Sicyoidochytrium, Thraustochytrium* et *Ulkenia.*

7. Procédé selon la revendication 6, **caractérisée en ce que** lesdits Thraustochytrides sont choisis parmi les espèces *Aurantiochytrium mangrovei* CCAP 4062/2 ; *Aurantiochytrium mangrovei* CCAP 4062/3 ; *Aurantiochytrium mangrovei* CCAP 4062/4 ; *Aurantiochytrium mangrovei,* CCAP 4062/5 ; *Aurantiochytrium mangrovei* CCAP 4062/6 ; *Aurantiochytrium mangrovei* CCAP 4062/1 ; *Schizochytrium sp.* 4087/3 ; *Schizochytrium sp*. CCAP 4087/1 ; *Schizochytrium sp.* CCAP 4087/4 ; *Schizochytrium sp.* CCAP 4087/5.

8. Biomasse de Thraustochytrides comprenant en poids par rapport au poids de la matière sèche, au moins 40% de protéines et au moins 20%, de matière grasse, la matière grasse ayant une teneur de 25 à 60% en acides gras polyinsaturés choisi parmi l'acide arachidonique (AA), l'acide docosahexaénoïque (DHA) et l'acide éicosapentaénoïque (EPA), sans protéines ni acides gras ajoutés susceptible d'être obtenue par le procédé selon l'une des revendications 1 à 7, à l'exclusion d'une biomasse des souches CNCM I-4469 et CNCM I-4702 déposées à la Collection Nationale de Culture de Microorganismes de l'Institut pasteur.

9. Biomasse selon la revendication 8, **caractérisée en ce qu'**elle comprend au moins 45% de protéines.

10. Biomasse selon l'une des revendications 8 ou 9, **caractérisée en ce qu'**elle comprend au moins 30% de matière grasse.

11. Biomasse selon l'une des revendications 8 à 10, **caractérisée en ce qu'**elle comprend entre 5 et 250 ppm de caroténoïdes.

12. Biomasse selon l'une quelconque des revendications 8 à 10, **caractérisée en ce que** lesdits Thraustochytrides sont d'un genre choisi dans le groupe comprenant *Aurantiochytrium, Aplanochytrium, Botryochytrium, Japonochytrium, Oblongichytrium, Parietichytrium, Schizochytrium, Sicyoidochytrium, Thraustochytrium* et *Ulkenia.*

13. Biomasse selon la revendication 11, **caractérisée en ce que** lesdits Thraustochytrides sont choisis parmi les espèces *Aurantiochytrium mangrovei* CCAP 4062/2 ; *Aurantiochytrium mangrovei* CCAP 4062/3 ; *Aurantiochytrium mangrovei* CCAP 4062/4 ; *Aurantiochytrium mangrovei,* CCAP 4062/5 ; *Aurantiochytrium mangrovei* CCAP 4062/6 ; *Aurantiochytrium mangrovei* CCAP 4062/1 ; *Schizochytrium sp.* 4087/3 ; *Schizochytrium sp.* CCAP 4087/1 ; *Schizochytrium sp.* CCAP 4087/4 ; *Schizochytrium sp*. CCAP 4087/5.

14. Utilisation d'une biomasse selon l'une des revendications 8 à 13, ou obtenue par le procédé selon l'une quelconque des revendications 1 à 7 dans l'alimentation humaine ou animale.

15. Biomasse selon l'une des revendications 8 à13 ou obtenue par le procédé selon l'une quelconque des revendications 1 à 7 pour son utilisation en thérapie.

## Patentansprüche

1. Verfahren zur Herstellung einer Thraustochytrium-Biomasse, von der eine im Wesentlichen mehrheitliche Menge nicht abgebaut ist und die Proteine und Fettsäuren umfasst, **dadurch gekennzeichnet, dass** sie, bezogen auf das Gewicht der Trockenmasse, mindestens 40 Gew.-% Proteine und mindestens 20 Gew.-% Fett umfasst, wobei das Fett einen Gehalt von 25 bis 60 % an mehrfach ungesättigten Fettsäuren aufweist, die ausgewählt sind aus Arachidonsäure (AA), Docosahexaensäure (DHA) und Eicosapentaensäure (EPA) ohne Zusatz von weder Proteinen noch Fettsäuren, **dadurch gekennzeichnet, dass** es Folgendes umfasst:
a. einen ersten Schritt der Kultur der Thraustochytriden, der im sogenannten "Batch"-Modus, im sogenannten "Fed-Batch"-Modus oder im kontinuierlichen Modus unter Heterotrophie-Bedingungen oder Mixotrophie-Bedingungen in einem definierten Kulturmedium, das eine Kohlenstoffquelle, eine Stickstoffquelle, eine Phosphorquelle und Salze umfasst, bei einer Temperatur von zwischen 24 °C und 35 °C und in Bedingungen ausgeführt wird, welche die Produktion von Proteinen mit einem Gehalt von mindestens 40 Gew.-% an Proteinen, bezogen auf das Gewicht der Trockenmasse, begünstigen, wobei der Schritt a) in zwei Teilschritte unterteilt ist, einen ersten Teilschritt a1) des Wachstums in dem geeigneten Kulturmedium, bis Gehalte an Kohlenstoffquelle in dem Medium von niedriger als 20 g/l erhalten werden, gefolgt von einem zweiten Teilschritt a2) der Herstellung, in welchem dem Kulturmedium gleichzeitig oder aufeinanderfolgend eine oder mehrere Lösungen zur Anreicherung an Kohlenstoffquelle, an Stickstoffquelle und an Phosphorquelle beigemengt werden, wobei der Gehalt an Kohlenstoffquelle zwischen 0 und 50 g/l gehalten wird, der Gehalt an Stickstoffquelle zwischen 0,5 und 5 g/l gehalten wird und der Gehalt an Phosphorquelle zwischen 0,5 und 5 g/l gehalten wird;
b. einen zweiten Schritt der Kultur in Heterotrophie- oder Mixotrophie-Bedingungen bei einer Temperatur von zwischen 18 °C und 25 °C und niedriger als diejenige von Schritt a) in Bedingungen, welche die Akkumulation von Fett bis zu mindestens 20 Gew.-%, bezogen auf das Gewicht der Trockenmasse, und bis zum Erhalt einer Kulturdichte an Trockenmasse von mindestens 40 g/l begünstigen;
c. einen dritten Schritt der Rückgewinnung der im zweiten Schritt erhaltenen Biomasse durch Trennung der Biomasse von dem Kulturmedium (der Ernte); und gegebenenfalls
d. einen vierten Schritt der Trocknung der im dritten Schritt rückgewonnenen Biomasse.

2. Verfahren nach Anspruch 1, **dadurch gekennzeichnet, dass** die Kultur mit einem Licht mit einer Wellenlänge von zwischen 435 und 475 nm beleuchtet wird.

3. Verfahren nach Anspruch 2, **dadurch gekennzeichnet, dass** die Kultur während mindestens den letzten 18 Stunden der Kultur beleuchtet wird.

4. Verfahren nach einem der Ansprüche 1 bis 3, **dadurch gekennzeichnet, dass** die Biomasse mindestens 45 % Proteine umfasst.

5. Verfahren nach einem der Ansprüche 1 bi 4, **dadurch gekennzeichnet, dass** sie mindestens 30 % Fett umfasst.

6. Verfahren nach einem der Ansprüche 1 bis 5, **dadurch gekennzeichnet, dass** die Thraustochytriden von einer Gattung sind, die ausgewählt wird aus der Gruppe, die *Aurantiochytrium, Aplanochytrium, Botryochytrium, Japonochytrium, Oblongichytrium, Parietichytrium, Schizochytrium, Sicyoidochytrium, Thraustochytrium* und *Ulkenia* umfasst.

7. Verfahren nach Anspruch 6, **dadurch gekennzeichnet, dass** die Thraustochytriden ausgewählt werden aus den Arten *Aurantiochytrium mangrovei* CCAP 4062/2; *Aurantiochytrium mangrovei* CCAP 4062/3; *Aurantiochytrium mangrovei* CCAP 4062/4; *Aurantiochytrium mangrovei* CCAP 4062/5; *Aurantiochytrium mangrovei* CCAP 4062/6; *Aurantiochytrium mangrovei* CCAP 4062/1; *Schizochytrium sp.* 4087/3; *Schizochytrium sp.* CCAP 4087/1; *Schizochytrium sp.* CCAP 4087/4; *Schizochytrium sp.* 4087/5.

8. Thraustochytrium-Biomasse, die, bezogen auf das Gewicht der Trockenmasse, mindestens 40 Gew.-% Proteine und mindestens 20 Gew.-% Fett umfasst, wobei das Fett einen Gehalt von 25 bis 60 % an mehrfach ungesättigten Fettsäuren aufweist, die ausgewählt sind aus Arachidonsäure (AA), Docosahexaensäure (DHA) und Eicosapentaensäure (EPA) ohne Zusatz von weder Proteinen noch Fettsäuren, die durch das Verfahren nach einem der Ansprüche 1 bis 7 erhalten werden kann, mit Ausnahme einer Biomasse der Stämme CNCM I-4469 und CNCM I-4702, die bei der Collection Nationale de Culture de Microorganismes des Institut Pasteur hinterlegt wurden.

9. Biomasse nach Anspruch 8, **dadurch gekennzeichnet, dass** sie mindestens 45 % Proteine umfasst.

10. Biomasse nach einem der Ansprüche 8 oder 9, **dadurch gekennzeichnet, dass** sie mindestens 30 % Fett umfasst.

11. Biomasse nach einem der Ansprüche 8 bis 10, **dadurch gekennzeichnet, dass** sie zwischen 5 und 250 ppm Carotinoide umfasst.

12. Biomasse nach einem der Ansprüche 8 bis 10, **dadurch gekennzeichnet, dass** die Thraustochytriden von einer Gattung sind, die ausgewählt ist aus der Gruppe, die *Aurantiochytrium, Aplanochytrium, Botryochytrium, Japonochytrium, Oblongichytrium, Parietichytrium, Schizochytrium, Sicyoidochytrium, Thraustochytrium* und *Ulkenia* umfasst.

13. Biomasse nach Anspruch 11, **dadurch gekennzeichnet, dass** die Thraustochytriden ausgewählt sind aus den Arten *Aurantiochytrium mangrovei* CCAP 4062/2; *Aurantiochytrium mangrovei* CCAP 4062/3; *Aurantiochytrium mangrovei* CCAP 4062/4; *Aurantiochytrium mangrovei* CCAP 4062/5; *Aurantiochytrium mangrovei* CCAP 4062/6; *Aurantiochytrium mangrovei* CCAP 4062/1; *Schizochytrium sp.* 4087/3; *Schizochytrium sp.* CCAP 4087/1; *Schizochytrium sp.* CCAP 4087/4; *Schizochytrium sp.* CCAP 4087/5.

14. Verwendung einer Biomasse nach einem der Ansprüche 8 bis 13, oder die nach dem Verfahren nach einem der Ansprüche 1 bis 7 erhalten wurde, bei der menschlichen oder tierischen Ernährung.

15. Biomasse nach einem der Ansprüche 8 bis 13, oder die nach einem der Ansprüche 1 bis 7 erhalten wurde, zur therapeutischen Verwendung.

## Claims

1. Method for preparing a biomass of Thraustochytrids, a substantially majority quantity of which is not degraded, comprising proteins and fatty acids, **characterised in that** it comprises, by weight relative to the weight of dry matter, at least 40% proteins and at least 20% fat, the fat having a content of 25 to 60% polyunsaturated fatty acids selected from arachidonic acid (AA), docosahexaenoic acid (DHA) and eicosapentaenoic acid (EPA), without added proteins or fatty acids, **characterised in that** it comprises:
a. a first step of culturing Thraustochytrids carried out in so-called "batch" mode, in so-called "fed batch" mode or in continuous mode under heterotrophic or mixotrophic conditions in a defined culture medium comprising a carbon source, a nitrogen source, a phosphorus source and salts, at a temperature between approximately 24°C and 35°C and under conditions for promoting the production of proteins at a content of at least 40% protein by weight relative to the weight of dry matter, step a) been divided into two sub-steps, a first growth sub-step a1) in the appropriate culture medium until obtaining carbon source contents in the medium less than 20 g/L, followed by a second, production sub-step a2) in which one or more solutions for enrichment in carbon source, nitrogen source and phosphorus source are simultaneously or successively added to the culture medium, the content of carbon source is maintained between 0 and 50 g/L, the content of nitrogen source is maintained between 0.5 and 5 g/L, and the content of phosphorus source is maintained between 0.5 and 5 g/L;
b. a second step of culturing under heterotrophic or mixotrophic conditions at a temperature between approximately 18°C and 25°C and less than that of step a), under conditions for promoting the accumulation of fat up to at least 20% by weight relative to the weight of dry matter, and until obtaining a culture density of at least 40 g/L dry matter;
c. a third step of recovering the biomass obtained in the second step, by separating said biomass from the culture medium (the harvest); and, if necessary
d. a fourth step of drying the biomass recovered in the third step.

2. Method according to claim 1, **characterised in that** the culture is illuminated with light of wavelength between 435 and 475 nm.

3. Method according to claim 2, **characterised in that** the culture is illuminated for at least the last 18 hours of culture.

4. Method according to one of claims 1 to 3, **characterised in that** the biomass comprises at least 45% proteins.

5. Method according to one of claims 1 to 4, **characterised in that** it comprises at least 30% fat.

6. Method according to any one of claims 1 to 5, **characterised in that** said Thraustochytrids are of a genus selected from the group comprising *Aurantiochytrium, Aplanochytrium, Botryochytrium, Japonochytrium, Oblongichytrium, Parietichytrium, Schizochytrium, Sicyoidochytrium, Thraustochytrium* and *Ulkenia.*

7. Method according to claim 6, **characterised in that** said Thraustochytrids are selected from the species *Aurantiochytrium mangrovei* CCAP 4062/2; *Aurantiochytrium mangrovei* CCAP 4062/3; *Aurantiochytrium mangrovei* CCAP 4062/4; *Aurantiochytrium mangrovei,* CCAP 4062/5; *Aurantiochytrium mangrovei* CCAP 4062/6; *Aurantiochytrium mangrovei* CCAP 4062/1; *Schizochytrium sp.* 4087/3; *Schizochytrium sp.* CCAP 4087/1; *Schizochytrium sp.* CCAP 4087/4; *Schizochytrium sp.* CCAP 4087/5.

8. Biomass of Thraustochytrids comprising, by weight relative to the weight of dry matter, at least 40% proteins and at least 20% fat, the fat having a content of 25 to 60% polyunsaturated fatty acids, chosen from arachidonic acid (AA), docosahexaenoic acid (DHA) and eicosapentaenoic acid (EPA), without added proteins or fatty acids, which can be obtained by the method according to one of claims 1 to 7, with the exclusion of a biomass of strains CNCM 1-4469 and CNCM I-4702 deposited in the Collection Nationale de Culture de Microorganismes of the Institut Pasteur.

9. Biomass according to claim 8, **characterised in that** it comprises at least 45% proteins.

10. Biomass according to one of claims 8 or 9, **characterised in that** it comprises at least 30% fat.

11. Biomass according to one of claims 8 to 10, **characterised in that** it comprises between 5 and 250 ppm carotenoids.

12. Biomass according to any one of claims 8 to 10, **characterised in that** said Thraustochytrids are of a genus selected from the group comprising *Aurantiochytrium, Aplanochytrium, Botryochytrium, Japonochytrium, Oblongichytrium, Parietichytrium, Schizochytrium, Sicyoidochytrium, Thraustochytrium* and *Ulkenia.*

13. Biomass according to claim 11, **characterised in that** said Thraustochytrids are selected from the species *Aurantiochytrium mangrovei* CCAP 4062/2; Aurantiochytrium mangrovei CCAP 4062/3; *Aurantiochytrium mangrovei* CCAP 4062/4; *Aurantiochytrium mangrovei,* CCAP 4062/5; *Aurantiochytrium mangrovei* CCAP 4062/6; *Aurantiochytrium mangrovei* CCAP 4062/1; *Schizochytrium sp.* 4087/3; *Schizochytrium sp.* CCAP 4087/1; Schizochytrium sp. CCAP 4087/4; *Schizochytrium sp.* CCAP 4087/5.

14. Use of a biomass according to one of claims 8 to 13, or obtained by the method according to any one of claims 1 to 7, in human or animal food.

15. Biomass according to one of claims 8 to 13, or obtained by the method according to any one of claims 1 to 7, for use thereof in therapy.
